# EUROPEAN PATENT SPECIFICATION

(11) **EP 4 122 912 B1**
(45) Date of publication and mention of the grant of the patent: **07.01.2026**
(21) Application number: 21898371.6
(22) Date of filing: 27.10.2021
(51) Int. Cl.: C07C 57/04

(54) **PROCESS FOR PRODUCING ACRYLIC ACID**
VERFAHREN ZUR HERSTELLUNG VON ACRYLSÄURE
PROCÉDÉ DE PRODUCTION D'ACIDE ACRYLIQUE

(30) Priority: 24.11.2020 KR 20200159107
(43) Date of publication of application: 25.01.2023
(73) Proprietor: LG Chem, Ltd., Seoul 07336 (KR)
(72) Inventor: KIM, Eunkyo, Daejeon 34122 (KR); KIM, Mi Kyung, Daejeon 34122 (KR); SHIN, Joon Ho, Daejeon 34122 (KR); KIM, Hyebin, Daejeon 34122 (KR)
(74) Representative: Goddar, Heinz J.
(86) International application number: PCT/KR2021/015174
(87) International publication number: WO 2022/114545

(56) References cited:
- WO-A1-2005/095320
- WO-A1-2016/026761
- JP-A- 2016 175 840
- JP-B2- 6 272 493
- KR-A- 20170 095 068
- KR-A- 20170 095 068
- US-A1- 2011 089 016

## Description

### [Technical Field]

The present application relates to a process for producing acrylic acid.

### [Background Art]

Acrylic acid has been generally produced through an oxidative dehydrogenation reaction of propylene, and demands on acrylic acid have increased as a raw material of super absorbent polymers, paints, and adhesives. Particularly, super absorbent polymers are used as hygiene products such as diapers.

So far, a considerable number of chemical products have been produced using raw materials derived from fossil raw materials such as coal or petroleum. However, using recyclable bio-derived resources as a carbon source has recently received attention as a substitute for existing fossil raw materials in terms of preventing global warming and protecting the environment. For example, development of methods using biomass resources including starch-based biomass such as corn or wheat, carbohydrate-based biomass such as sugar cane, and cellulose-based biomass such as residue of rapeseed or rice straw as a raw material has been attempted.

In order words, studies on breaking from existing petrochemical-based manufacturing processes and producing chemical products based on environmental-friendly raw materials to obtain excellent properties in terms of environmental protection while obtaining sustainability are recently in progress.

One type of reactions producing other chemical products from lactic acid may include a gas-phase reaction in which a raw material including lactic acid is evaporated and brought into contact with a catalyst in a gaseous state to obtain a product. For example, as a technology of producing acrylic acid using lactic acid, a gas-phase dehydration reaction using a solid catalyst is known, and the dehydration reaction of lactic acid is mainly studied as a gas-phase reaction.

Lactic acid is a substance that polymerizes as an esterification reaction occurs in a liquid phase without a catalyst in the absence of water, and reacts as a lactic acid oligomer as lactic acid is concentrated and a concentration thereof increases. Dehydration occurs as lactic acid is oligomerized, and an oligomerization reaction of lactic acid occurs as the lactic acid is concentrated without water.

When the lactic acid oligomer is introduced to a reactor for producing acrylic acid, fouling occurs in the reactor and the reaction yield decreases, and therefore, studies on a method to decrease the content of lactic acid oligomer for producing acrylic acid is in progress.

Among them, lactic acid is introduced to a vaporizer in an aqueous lactic acid solution state in order to reduce the lactic acid oligomer content, however, water with a low boiling point is vaporized first and then lactic acid is vaporized in the vaporizer, and a problem of producing a lactic acid oligomer still occurs as the lactic acid is concentrated in the liquid phase during the vaporization.

In addition, a distillation tower that separates using a boiling point difference so as not to include an oligomer in a vaporized aqueous lactic acid solution may be used, however, an oligomerization reaction of lactic acid occurs herein as well concentrating the lactic acid oligomer, and a problem of increasing a temperature of a low portion of the distillation tower occurs.

Accordingly, in view of the above, studies to decrease a lactic acid oligomer content and increasing a produced acrylic acid yield are in progress.

### Prior Art Documents

### Patent Documents

(Patent Document 1) International Patent Application Laid-Open Publication No. 2005-095320

KR 2017 0095068 A discloses a method of producing acrylic acid by using hydroxypropionic acid, the method comprising the steps of: (a) providing a reactive distillation column which includes a reaction section and an absorption section located above the reaction section, and in which the reaction section and the absorption section each have one or more stages; (b) supplying hydroxypropionic acid to the absorption section, and supplying an acid catalyst to the reaction section to produce acrylic acid and water in the reaction section; (c) moving water produced in the step (b) in the form of vapor together with a portion of the acrylic acid to the absorption section, and absorbing the portion of the acrylic acid by hydroxypropionic acid supplied to the absorption section in the step (b); and (d) discharging water from which the acrylic acid has been removed in the step (c) to the top of the reactive distillation column, and discharging the acrylic acid absorbed by hydroxypropionic acid together with the acrylic acid produced in the reaction section to the bottom of the reactive distillation column.

### [Disclosure]

### [Technical Problem]

The present application is directed to providing a process for producing acrylic acid.

### [Technical Solution]

One embodiment of the present application provides a process for producing acrylic acid, the process including a step of supplying a lactic acid raw material to an upper portion of a distillation tower; a step of supplying water to a lower portion of the distillation tower; a step of supplying a first lactic acid vapor vaporized in the distillation tower to a reactor; and a step of supplying a second aqueous lactic acid solution not vaporized in the distillation tower to a middle portion of the distillation tower.

Further embodiments are disclosed in the dependent claims.

### [Advantageous Effects]

A process for producing acrylic acid according to one embodiment of the present application uses a distillation tower that separates using a boiling point difference, and in order to particularly resolve a problem of increasing a temperature of a lower portion of the distillation tower during the distillation tower operation, water and a concentrated lactic acid raw material are separately supplied instead of supplying to the distillation tower as an aqueous lactic acid solution form. Accordingly, by supplying water to a lower portion of the distillation tower and thereby decomposing some of lactic acid oligomer concentrated in the lower portion to lactic acid, the temperature of the lower portion of the distillation tower can be lowered.

Particularly, lactic acid is thermally decomposed at a high temperature (approximately 200°C to 250°C or higher) and has a problem of producing by-products such as propionic acid, however, by supplying water separately to a lower portion of the distillation tower as above, a lower portion temperature of the distillation tower and a temperature of the distillation tower can be lowered, which suppresses generation of by-products caused by thermal decomposition of lactic acid.

In addition, the process for producing acrylic acid according to the present application uses a distillation tower instead of a vaporizer, and is capable of preventing formation of a lactic acid oligomer during vaporization of an aqueous lactic acid solution, and, by introducing a concentrated lactic acid raw material to the distillation tower as it is, is capable of producing acrylic acid without a separate dilution device.

### [Description of Drawings]

FIG. 1 is a schematic diagram of a process for producing acrylic acid according to one embodiment of the present application.
FIG. 2 is a schematic diagram of a process for producing acrylic acid according to Comparative Example 1 of the present application.
FIG. 3 is a schematic diagram of a process for producing acrylic acid according to Comparative Example 2 of the present application.

### <Reference Numeral>

100: Distillation Tower
200: Heat Exchanger
1: Liquid Aqueous Lactic Acid Solution
2: Water
3: First Lactic Acid Vapor
4: Second Aqueous Lactic Acid Solution

### [Mode for Disclosure]

Hereinafter, the present specification will be described in more detail.

In the present specification, a description of a certain part "including" certain constituents means capable of further including other constituents, and does not exclude other constituents unless particularly stated on the contrary.

In the present specification, 'p to q' means a range of 'greater than or equal to p and less than or equal to q'.

Hereinafter, embodiments of the present disclosure will be described in detail with reference to accompanying drawings so that those having common knowledge in the art may readily implement the present disclosure.

One embodiment of the present application provides a process for producing acrylic acid, the process including a step of supplying a lactic acid raw material to an upper portion of a distillation tower; a step of supplying water to a lower portion of the distillation tower; a step of supplying a first lactic acid vapor vaporized in the distillation tower to a reactor; and a step of supplying a second aqueous lactic acid solution not vaporized in the distillation tower to a middle portion of the distillation tower.

The process for producing acrylic acid according to one embodiment of the present application uses a distillation tower that separates using a boiling point difference, and in order to particularly resolve a problem of increasing a temperature of a lower portion of the distillation tower during the distillation tower operation, water is supplied to a lower portion of the distillation tower instead of supplying to the distillation tower as an aqueous lactic acid solution form, and, by decomposing some of lactic acid oligomer concentrated in the lower portion to lactic acid, the temperature of the lower portion of the distillation tower may be lowered.

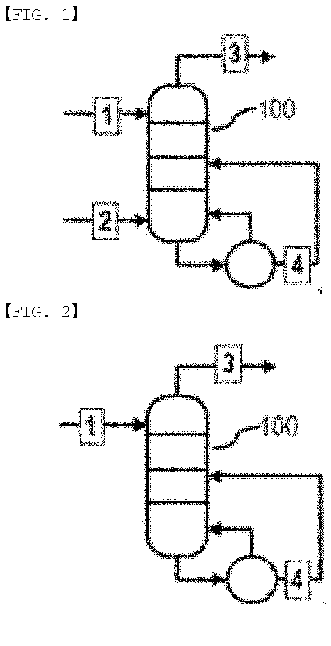

FIG. 1 is a schematic diagram of a process for producing acrylic acid according to one embodiment of the present application. Specifically, FIG. 1 uses a 4-level distillation tower (100), and a step of supplying a lactic acid raw material to a distillation tower (100) (1), a step of supplying water to a lower portion of the distillation tower (2), a step of supplying a first lactic acid vapor vaporized in the distillation tower to a reactor (3), and a step of supplying a second aqueous lactic acid solution not vaporized in the distillation tower to a middle portion of the distillation tower (4) may be specifically identified.

In one embodiment of the present application, the lactic acid raw material includes water; lactic acid; and a lactic acid oligomer, and may include the water in 20 parts by weight or less based on 100 parts by weight of the lactic acid raw material.

In the present application, the lactic acid is an organic compound having an asymmetric carbon atom to which four atomic groups of a carboxyl group, a hydroxyl group, a methyl group and hydrogen bond, includes both D-lactic acid and L-lactic acid, and may mean a single lactic acid monomer.

In the present application, a lactic acid oligomer means a material obtained by lactic acid reacting to each other to form a dimer, or a trimer, and the lactic acid oligomer may mean a dimer to a 100-mer of lactic acid.

Lactic acid is a substance that polymerizes through an esterification reaction in a liquid phase without a catalyst even in the absence of water, and substances formed through a polymerization reaction of lactic acid may all be expressed as a lactic acid oligomer.

In one embodiment of the present application, the lactic acid raw material includes water; lactic acid; and a lactic acid oligomer, and may include the water in 20 parts by weight or less, preferably 18 parts by weight or less and more preferably 13 parts by weight or less based on 100 parts by weight of the lactic acid raw material.

In one embodiment of the present application, the lactic acid raw material includes water; lactic acid; and a lactic acid oligomer, and may include the water in 0 parts by weight or greater, preferably 1 parts by weight or greater and more preferably 1.5 parts by weight or greater based on 100 parts by weight of the lactic acid raw material.

The process for producing acrylic acid according to the present application introduces a concentrated lactic acid raw material to a distillation tower as it is, and by minimizing a water content in the lactic acid raw material as in the above-mentioned range, acrylic acid may be produced without a separate lactic acid dilution device.

In the process for producing acrylic acid provided in one embodiment of the present application, the upper portion of the distillation tower has a temperature of higher than or equal to 150°C and lower than or equal to 200°C, and the lower portion of the distillation tower has a temperature of higher than or equal to 200°C and lower than or equal to 250°C.

In another embodiment, the upper portion of the distillation tower may have a temperature of higher than or equal to 150°C and lower than or equal to 200°C, preferably higher than or equal to 155°C and lower than or equal to 190°C, more preferably higher than or equal to 160°C and lower than or equal to 185°C, and most preferably higher than or equal to 170°C and lower than or equal to 180°C.

In another embodiment, the lower portion of the distillation tower may have a temperature of higher than or equal to 200°C and lower than or equal to 250°C, preferably higher than or equal to 210°C and lower than or equal to 245°C, more preferably higher than or equal to 220°C and lower than or equal to 245°C, and most preferably higher than or equal to 230°C and lower than or equal to 245°C.

Lactic acid is thermally decomposed at a high temperature (approximately 200°C to 250°C or higher) and has a problem of producing by-products such as propionic acid, however, the present application is capable of lowering a lower portion temperature of the distillation tower and a whole temperature of the distillation tower by supplying water separately to the lower portion of the distillation tower as above, and generation of by-products caused by thermal decomposition of lactic acid may be suppressed by the upper portion and the lower portion of the distillation tower having the above-mentioned temperature ranges.

In the process for producing acrylic acid provided in one embodiment of the present application, a ratio of the lactic acid:lactic acid oligomer in the first lactic acid vapor is from 100:0 to 95:5.

The first lactic acid vapor is a material obtained by separating the lactic acid raw material introduced to distillation tower using a boiling point difference, and the first lactic acid vapor may include water; lactic acid; and a lactic acid oligomer.

In another embodiment, a ratio of the lactic acid:lactic acid oligomer in the first lactic acid vapor may satisfy a ratio of 100:0 to 95:5, and most preferably 100:0.

The process for producing acrylic acid according to the present application uses a distillation tower instead of a vaporizer, and is capable of preventing formation of a lactic acid oligomer during vaporization of the aqueous lactic acid solution, and in producing gas-phase acrylic acid, an occurrence of fouling in the reactor may be minimized by reducing the oligomer content, and the reaction yield may be maximized.

In the process for producing acrylic acid provided in one embodiment of the present application, a ratio of the lactic acid:lactic acid oligomer in the second aqueous lactic acid solution is from 1:99 to 20:80.

The second aqueous lactic acid solution includes an oligomer concentrated in the lower portion of the distillation tower without being vaporized in the distillation tower, and in the present application, the concentrated oligomer may be decomposed to lactic acid again by directly introducing water to the lower portion of the distillation tower, which may directly lower a temperature of the lower portion of the distillation tower.

In addition, by supplying the stream of the second aqueous lactic acid solution again to a middle portion of the distillation tower, the distillation tower may be operated with minimized lactic acid loss.

In one embodiment of the present application, the distillation tower has only a reboiler without a condenser, and may mean an evaporator for heating and evaporating a liquid rich in components with a high boiling point extracted at the bottom of the distillation tower, returning the generated vapor back to the bottom of the distillation tower, and extracting the remaining liquid as an effluent, and distillation towers commonly used in the art may be used without limit.

In the process for producing acrylic acid provided in one embodiment of the present application, the distillation tower is formed to have 3 levels to 7 levels with the upper portion being a first level, the middle portion being a level between the upper portion and the lower portion, and the lower portion being a last level.

The distillation tower according to the present application may be formed to have 5 levels, and the number of levels of the distillation tower is not limited such as 10 levels or 15 levels, and in this case, the upper portion means an uppermost level of the levels of the distillation tower, the lower portion may mean a lowermost level of the levels of the distillation tower, and the middle portion of the distillation tower may mean all levels other than the upper portion and the lower portion.

In the process for producing acrylic acid provided in one embodiment of the present application, the distillation tower has an inner pressure of greater than or equal to 0.1 bar and less than or equal to 2.0 bar.

In another embodiment, the inner pressure of the distillation tower may satisfy a range of greater than or equal to 0.1 bar and less than or equal to 2.0 bar, and preferably greater than or equal to 0.3 bar and less than or equal to 1.8 bar.

By the inner pressure of the distillation tower satisfying the above-mentioned range as above, decomposition of lactic acid may be minimized due to a suitable temperature in a vaporizer, and by reducing a pressure difference with the reactor later on, capacity of a compressor used may be set to a proper range.

Lactic acid-based is highly corrosive and exhibits significant corrosiveness particularly at a temperature of higher than 200°C. Accordingly, materials of the distillation tower and the reactor preferably have some degree of corrosion resistance to lactic acid-based. Examples of the materials having corrosion resistance to lactic acid-based may include austenite-based stainless, ferrite-based stainless, duplex stainless, nickel alloys, titanium, zirconium, tantalum, titanium alloys, gold, and platinum. The vapor composition including lactic acid obtained as above may be brought into contact with a catalyst and converted into other useful chemical products. Examples of products from lactic acid-based may include acrylic acid, and pyruvic acid.

In the process for producing acrylic acid provided in one embodiment of the present application, the first lactic acid vapor includes water; lactic acid; and a lactic acid oligomer, and includes the lactic acid and the lactic acid oligomer in greater than or equal to 5 parts by weight and less than or equal to 80 parts by weight based on 100 parts by weight of the first lactic acid vapor.

In another embodiment, the first lactic acid vapor includes water; lactic acid; and a lactic acid oligomer, and may include the lactic acid and the lactic acid oligomer in greater than or equal to 5 parts by weight and less than or equal to 80 parts by weight, preferably greater than or equal to 10 parts by weight and less than or equal to 75 parts by weight, and more preferably greater than or equal to 30 parts by weight and less than or equal to 70 parts by weight based on 100 parts by weight of the first lactic acid vapor.

By the first lactic acid vapor having a lactic acid-based content range as above, effects of the process for producing acrylic acid are excellent by having a proper introduced amount supplied to the reactor later on, and excellent economic feasibility is obtained in the process by introducing a proper amount of water to the reactor.

In the process for producing acrylic acid provided in one embodiment of the present application, acrylic acid is produced through, after the step of supplying a first lactic acid vapor vaporized in the distillation tower to a reactor, a dehydration reaction of the first lactic acid vapor.

In other words, the process for producing acrylic acid according to the present disclosure breaks from existing petrochemical-based manufacturing processes and produces acrylic acid based on lactic acid, an environmental-friendly raw material, and as a result, excellent properties are obtained in terms of environmental protection while obtaining sustainability. Particularly, by minimizing a content of lactic acid oligomer introduced to a reactor through using a distillation tower and separately introducing water to a lower portion thereof, an acrylic acid yield is maximized, and an occurrence of fouling in the reactor is suppressed.

The production process of the present disclosure is particularly useful for synthesizing acrylic acid, and specifically, the vapor composition including lactic acid obtained in the present disclosure may be brought into contact with a dehydration catalyst to prepare acrylic acid. A produced reaction gas is collected and liquefied by cooling or brining into contact with a collection liquid, and after going through a purification process such as extraction, distillation or crystallization, high purity acrylic acid may be obtained. Produced acrylic acid is widely used as a raw material of absorbent polymers, paints, or adhesives.

Hereinafter, examples of the present disclosure will be described in detail so that those having common knowledge in the art may readily implement the present disclosure.

### <Preparation Example>

The following examples and comparative examples were simulated by Aspen Plus of Aspen Technology Inc..

### Example 1

A distillation tower was formed to have 5 levels having only a reboiler without a condenser. A purified concentrated lactic acid raw material was introduced to a first level (uppermost level) of the distillation tower without a step of dilution. In order to lower an operation temperature by decomposing a lactic acid oligomer at a lower portion of the distillation tower, water was introduced to a fifth level (lowermost level) of the distillation tower.

A stream of the upper portion was obtained as a gas phase (first lactic acid vapor) and used as a feed for a reactor. A stream coming out of the lower portion (second aqueous lactic acid solution) was re-introduced back to the distillation tower to operate without lactic acid loss, and the re-introduced level for the stream of the lower portion was the second level in the operation.

The distillation tower used had an inner pressure of 1.5 bar.

The operation process of Example 1 may be identified in FIG. 1, and as illustrated in FIG. 1, flow rates of the liquid aqueous lactic acid solution (1), water (2), the first lactic acid vapor (3) and the second aqueous lactic acid solution (4), and a composition of each stream are as shown in the following Table 1.

**[Table 1]**

| Stream No. | | 1 | 2 | 3 | 4 |
|---|---|---|---|---|---|
| Flow Rate (kg/hr) | | 440 | 560 | 1000 | 500 |
| Compos ition | Water | 0.124 | 1.000 | 0.600 | 0.005 |
| | Lactic Acid | 0.602 | 0.000 | 0.400 | 0.147 |
| | Lactic Acid Oligomer | 0.274 | 0.000 | 0.000 | 0.848 |

### Comparative Example 1

An operation was conducted in the same manner as in Example 1 except that the lactic acid raw material and water were not separately introduced, and the purified concentrated lactic acid raw material was diluted in water to prepare an aqueous solution having 40% of the lactic acid raw material (lactic acid and lactic acid oligomer), and the aqueous solution was introduced to a first level (uppermost level) of the distillation tower, and water was not introduced to a fifth level (lowermost level) of the distillation tower.

The distillation tower used had an inner pressure of 1.5 bar.

Specifically, the operation manner may be identified in FIG. 2. The concentrated lactic acid raw material diluted in water to prepare an aqueous solution having 40% of the lactic acid raw material (lactic acid and lactic acid oligomer) was introduced to a stream No. 1, and it is identified that the process of introducing water as the stream No. 2 of FIG. 1 is not conducted. As illustrated in FIG. 2, flow rates of the liquid aqueous lactic acid solution (1), the first lactic acid vapor (3) and the second aqueous lactic acid solution (4), and a composition of each stream are as shown in the following Table 2.

**[Table 2]**

| Stream No. | | 1 | 3 | 4 |
|---|---|---|---|---|
| Flow Rate (kg/hr) | | 1000 | 1000 | 500 |
| Compos ition | Water | 0.602 | 0.602 | 0.000 |
| | Lactic Acid | 0.380 | 0.380 | 0.000 |
| | Lactic Acid Oligomer | 0.018 | 0.018 | 1.000 |

### Comparative Example 2

A purified concentrated lactic acid raw material was diluted in water to prepare an aqueous solution having 40% of the lactic acid raw material (lactic acid and lactic acid oligomer). The aqueous solution was introduced to a heat exchanger, 3% thereof was vaporized in the heat exchanger, and the liquid not vaporized was recirculated again. The flow rate of the recirculated liquid was approximately 50 times that of the liquid aqueous lactic acid solution, and the recirculated liquid was introduced to the heat exchanger after meeting with the diluted aqueous lactic acid solution. The stream of the vapor-phase lactic acid vaporized in the heat exchanger was used as reactor feed.

The heat exchanger used had a pressure of 1.5 bar and a temperature of 217°C.

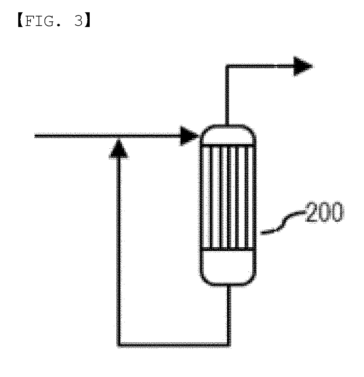

Specifically, the operation manner may be identified in FIG. 3, and it is identified that, unlike Example 1 and Comparative Example 1, operation using a heat exchanger (200) instead of using a distillation tower is identified.

The composition and the content of the lactic acid vapor (first lactic acid vapor) supplied to the final reactor feed through each of the processes of Example 1, Comparative Example 1 and Comparative Example 2 are described in the following Table 3, and a temperature of the reboiler of the distillation tower used is also described in the following Table 3.

**[Table 3]**

| | | Example 1 | Comparative Example 1 | Comparative Example 2 |
|---|---|---|---|---|
| Lactic Acid Vapor Composition (Reactor Feed) | Water | 60.0% | 60.2% | 60.6% |
| | Lactic Acid | 40.0% | 38.0% | 33.9% |
| | Lactic Acid Oligomer | 0.0% | 1.8% | 5.4% |
| Reboiler | °C | 242 | 359 | - |
| Temperature (Temperature of Lower Portion of Distillation Tower) | | | | |
| Temperature of Upper Portion of Distillation Tower | °C | 173 | 200 | - |

In Table 3, the composition and the content of final lactic acid vapor supplied to the reactor feed may be identified, and particularly, it was identified that, by Comparative Example 1 separating the lactic acid oligomer using a distillation tower, a ratio of the lactic acid oligomer in the lactic acid raw material decreased to a 3% range with the lactic acid being approximately 38 wt% and the lactic acid oligomer being 1.8 wt%. However, as the reboiler temperature of the distillation tower increased close to 360°C, the lactic acid was very likely to be thermally decomposed (lactic acid is thermally decomposed at a temperature of 200°C to 250°C or higher), and loss of lactic acid supplied to the reactor feed later on was identified.

In Example 1 of Table 3, introducing water to a lowermost portion of the distillation tower performs a role of decomposing the lactic acid oligomer concentrated in the lower portion of the distillation tower, which decreases a temperature of the lower portion of the distillation tower, and it was identified that the reboiler temperature was lowered to approximately 240°C whereas it was approximately 360°C in Comparative Example 1, and in addition, there was almost no lactic acid oligomer in the composition of the lactic acid vapor in the reactor feed. In addition, it was identified that, although the flow rates of the introduced aqueous lactic acid solution (1) and water (2) were the same compared to in Comparative Example 1, there was a difference in that the water (2) was introduced to a lowermost portion of the distillation tower.

In Comparative Example 2 of Table 3, the lactic acid raw material (lactic acid and lactic acid oligomer) was approximately 39 wt%, however, lactic acid was just 34 wt% therein based on the lactic acid raw material, and the rest was the lactic acid oligomer, and it was identified that a problem of having a high lactic acid oligomer content occurred with a ratio of the lactic acid oligomer being approximately 14% in the lactic acid raw material.

In other words, the process for producing acrylic acid according to one embodiment of the present application uses a distillation tower that separates using a boiling point difference, and in order to particularly resolve a problem of increasing a temperature of a lower portion of the distillation tower during the distillation tower operation, water and a concentrated lactic acid raw material are separately supplied instead of supplying to the distillation tower as an aqueous lactic acid solution form, and accordingly, it was identified that, by supplying water to a lower portion of the distillation tower and thereby decomposing some of lactic acid oligomer concentrated in the lower portion to lactic acid, the temperature of the lower portion of the distillation tower may be lowered.

In addition, the process for producing acrylic acid according to the present application uses a distillation tower instead of a vaporizer, and it was identified that the process was able to prevent formation of the lactic acid oligomer during vaporization of the aqueous lactic acid solution, and, by introducing the concentrated lactic acid raw material to the distillation tower as it is, was able to produce acrylic acid without a separate dilution device.

## Claims

1. A process for producing acrylic acid, the process comprising:
a step of supplying a lactic acid raw material to an upper portion of a distillation tower;
a step of supplying water to a lower portion of the distillation tower;
a step of supplying a first lactic acid vapor vaporized in the distillation tower to a reactor; and
a step of supplying a second aqueous lactic acid solution not vaporized in the distillation tower to a middle portion of the distillation tower.

2. The process for producing acrylic acid of Claim 1, wherein the lactic acid raw material includes water; lactic acid; and a lactic acid oligomer, and includes the water in 20 parts by weight or less based on 100 parts by weight of the lactic acid raw material.

3. The process for producing acrylic acid of Claim 1, wherein the upper portion of the distillation tower has a temperature of higher than or equal to 150°C and lower than or equal to 200°C, and the lower portion of the distillation tower has a temperature of higher than or equal to 200°C and lower than or equal to 250°C.

4. The process for producing acrylic acid of Claim 1, wherein a ratio of the lactic acid:lactic acid oligomer in the first lactic acid vapor is from 100:0 to 95:5.

5. The process for producing acrylic acid of Claim 1, wherein a ratio of the lactic acid:lactic acid oligomer in the second aqueous lactic acid solution is from 1:99 to 20:80.

6. The process for producing acrylic acid of Claim 1, wherein the distillation tower has an inner pressure of greater than or equal to 0.1 bar and less than or equal to 2.0 bar.

7. The process for producing acrylic acid of Claim 1, wherein the distillation tower is formed to have 3 levels to 7 levels with the upper portion being a first level, the middle portion being a level between the upper portion and the lower portion, and the lower portion being a last level.

8. The process for producing acrylic acid of Claim 1, wherein the first lactic acid vapor includes water; lactic acid; and a lactic acid oligomer, and includes the lactic acid and the lactic acid oligomer in greater than or equal to 5 parts by weight and less than or equal to 80 parts by weight based on 100 parts by weight of the first lactic acid vapor.

9. The process for producing acrylic acid of Claim 1, which produces acrylic acid through, after the step of supplying a first lactic acid vapor vaporized in the distillation tower to a reactor, a dehydration reaction of the first lactic acid vapor.

## Patentansprüche

1. Verfahren zur Herstellung von Acrylsäure, wobei das Verfahren umfasst:
einen Schritt des Zuführens eines Milchsäurerohmaterials zu einem oberen Abschnitt eines Destillationsturms;
einen Schritt des Zuführens von Wasser zu einem unteren Abschnitt des Destillationsturms;
einen Schritt des Zuführens eines ersten Milchsäuredampfes, der in dem Destillationsturm verdampft wird, zu einem Reaktor; und
einen Schritt des Zuführens einer zweiten wässrigen Milchsäurelösung, die nicht in dem Destillationsturm verdampft wird, zu einem mittleren Abschnitt des Destillationsturms.

2. Verfahren zur Herstellung von Acrylsäure nach Anspruch 1, wobei das Milchsäurerohmaterial Wasser; Milchsäure; und ein Milchsäureoligomer umfasst, und das Wasser in 20 Gewichtsteilen oder weniger basierend auf 100 Gewichtsteilen des Milchsäurerohmaterials umfasst.

3. Verfahren zur Herstellung von Acrylsäure nach Anspruch 1, wobei der obere Abschnitt des Destillationsturms eine Temperatur von höher als oder gleich 150 °C und niedriger als oder gleich 200 °C aufweist, und der untere Abschnitt des Destillationsturms eine Temperatur von höher als oder gleich 200 °C und niedriger als oder gleich 250 °C aufweist.

4. Verfahren zur Herstellung von Acrylsäure nach Anspruch 1, wobei ein Verhältnis des Milchsäure:Milchsäure-Oligomers in dem ersten Milchsäuredampf von 100:0 bis 95:5 beträgt.

5. Verfahren zur Herstellung von Acrylsäure nach Anspruch 1, wobei ein Verhältnis des Milchsäure:Milchsäure-Oligomers in der zweiten wässrigen Milchsäurelösung von 1:99 bis 20:80 beträgt.

6. Verfahren zur Herstellung von Acrylsäure nach Anspruch 1, wobei der Destillationsturm einen Innendruck von höher als oder gleich 0,1 bar und niedriger als oder gleich 2,0 bar aufweist.

7. Verfahren zur Herstellung von Acrylsäure nach Anspruch 1, wobei der Destillationsturm so ausgebildet ist, dass er 3 Ebenen bis 7 Ebenen aufweist, wobei der obere Abschnitt eine erste Ebene ist, der mittlere Abschnitt eine Ebene zwischen dem oberen Abschnitt und dem unteren Abschnitt ist, und der untere Abschnitt eine letzte Ebene ist.

8. Verfahren zur Herstellung von Acrylsäure nach Anspruch 1, wobei der erste Milchsäuredampf Wasser; Milchsäure; und ein Milchsäureoligomer umfasst, und die Milchsäure und das Milchsäureoligomer in höher als oder gleich 5 Gewichtsteilen und niedriger als oder gleich 80 Gewichtsteilen basierend auf 100 Gewichtsteilen des ersten Milchsäuredampfes umfasst.

9. Verfahren zur Herstellung von Acrylsäure nach Anspruch 1, das Acrylsäure durch, nach dem Schritt des Zuführens eines ersten Milchsäuredampfes, der in dem Destillationsturm verdampft wird, zu einem Reaktor, eine Dehydratisierungsreaktion des ersten Milchsäuredampfes herstellt.

## Revendications

1. Procédé de production d'acide acrylique, le procédé comprenant :
une étape de fourniture d'une matière première d'acide lactique à une partie supérieure d'une tour de distillation ;
une étape de fourniture d'eau à une partie inférieure de la tour de distillation ;
une étape de fourniture d'une première vapeur d'acide lactique vaporisée dans la tour de distillation à un réacteur ; et
une étape de fourniture une deuxième solution d'acide lactique aqueuse non vaporisée dans la tour de distillation à une partie médiane de la tour de distillation.

2. Procédé de production d'acide acrylique selon la revendication 1, dans lequel la matière première d'acide lactique inclut de l'eau ; de l'acide lactique ; et un oligomère d'acide lactique, et inclut de l'eau dans une quantité égale ou inférieure à 20 parties en poids sur la base de 100 parties en poids de la matière première d'acide lactique.

3. Procédé de production d'acide acrylique selon la revendication 1, dans lequel la partie supérieure de la tour de distillation a une température égale ou supérieure à 150°C et égale ou inférieure à 200°C, et la partie inférieure de la tour de distillation a une température égale ou supérieure à 200°C et égale ou inférieure à 250°C.

4. Procédé de production d'acide acrylique selon la revendication 1, dans lequel un rapport de l'acide lactique à l'oligomère d'acide lactique dans la première vapeur d'acide lactique est de 100:0 à 95:5.

5. Procédé de production d'acide acrylique selon la revendication 1, dans lequel un rapport de l'acide lactique à l'oligomère d'acide lactique dans la deuxième solution d'acide lactique aqueuse est de 1:99 à 20:80.

6. Procédé de production d'acide acrylique selon la revendication 1, dans lequel la tour de distillation a une pression interne égale ou supérieure à 0,1 bar et égale ou inférieure à 2,0 bar.

7. Procédé de production d'acide acrylique selon la revendication 1, dans lequel la tour de distillation est formée pour avoir 3 niveaux à 7 niveaux avec la partie supérieure étant un premier niveau, la partie médiane étant un niveau entre la partie supérieure et la partie inférieure, et la partie inférieure étant un dernier niveau.

8. Procédé de production d'acide acrylique selon la revendication 1, dans lequel la première vapeur d'acide lactique inclut de l'eau ; de l'acide lactique ; et un oligomère d'acide lactique, et inclut l'acide lactique et l'oligomère d'acide lactique dans une quantité égale ou supérieure à 5 parties en poids et égale ou inférieure à 80 parties en poids sur la base de 100 parties en poids de la première vapeur d'acide lactique.

9. Procédé de production d'acide acrylique selon la revendication 1, qui produit de l'acide acrylique par le biais de, après l'étape de fourniture d'une première vapeur d'acide lactique vaporisée dans la tour de distillation à un réacteur, une réaction de déshydratation de la première vapeur d'acide lactique.
